(19) Europäisches Patentamt
European Patent Office
Office européen des brevets

(11) Numéro de publication: **0 287 436 A1**

# (12) DEMANDE DE BREVET EUROPEEN

(21) Numéro de dépôt: **88400839.2**

(22) Date de dépôt: **07.04.88**

(51) Int. Cl.4: **C 07 D 257/02**
C 08 F 12/32, B 01 J 45/00

(30) Priorité: **10.04.87 FR 8705117**

(43) Date de publication de la demande: **19.10.88 Bulletin 88/42**

(84) Etats contractants désignés: **AT BE CH DE ES FR GB GR IT LI LU NL SE**

(71) Demandeur: **CENTRE NATIONAL DE LA RECHERCHE SCIENTIFIQUE**
**15, quai Anatole France**
**F-75007 Paris (FR)**

(72) Inventeur: **Handel, Henri**
**30, rue E. Corblère**
**F-29200 Brest (FR)**

**Chaumeil, Hélène**
**6, rue Francis Garnier**
**F-29200 Brest (FR)**

(74) Mandataire: **Ahner, Francis et al**
**CABINET REGIMBEAU 26, avenue Kléber**
**F-75116 Paris (FR)**

## (54) Tétramines cycliques monofonctionnalisées, leur procédé de préparation, leurs polymères et utilisation de ces polymères.

(57) L'invention concerne un procédé de préparation de tétramines cycliques monofonctionnalisées de formule I suivante :

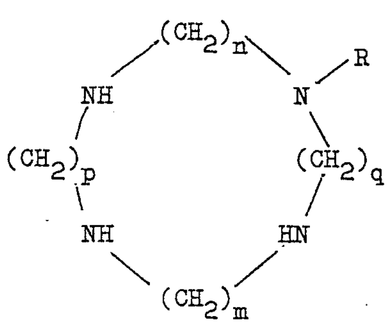

(I)

dans laquelle :

$n = m = p = q = 2$ ou
$n = m = 2$ ; $p = q = 3$ ou
$n = 2$ ; $m = p = q = 3$ ou
$m = 2$ ; $n = p = q = 3$ ou
$n = m = p = q = 3$ ou
$n = m = 3$ ; $p = q = 4$,

R représente un radical organique saturé ou non, notamment polymérisable, les tétramines cycliques obtenues, leur polymères et l'utilisation de ces polymères comme résine échangeuse d'ions.

EP 0 287 436 A1

**Description**

**TETRAMINES CYCLIQUES MONOFONCTIONNALISEES, LEUR PROCEDE DE PREPARATION, LEURS POLYMERES ET UTILISATION DE CES POLYMERES.**

La présente invention concerne un procédé pour la préparation de tétramines cycliques ou tétraazacycloalcanes monofonctionnelles, les produits obtenus, éventuellement polymérisés, et l'utilisation de ces produits polymérisés comme résines échangeuses d'ions.

La chimie des coordinats macrocycliques et macropolycycliques connaît depuis plusieurs années un développement considérable comme en témoigne l'aboncance des articles consacrés à ce sujet (1). Les complexants les plus courants de cette famille sont des hydrocarbures cycliques oxygénés, azotés ou mixtes (oxygénés et azotés). L'importance de cette classe de composés réside principalement dans l'aptitude que possède ce type de ligand à former sélectivement des complexes stables avec bon nombre de cations métalliques.

Alors que les complexants oxygénés (par exemple les éthers-couronnes) ou mixtes (par exemple les cryptands), s'associent essentiellement aux ions alcalins et alcalino-terreux, les tétramines cycliques se distinguent par le fait qu'elles sont capables de former des complexes extrêmement stables avec de nombreux cations de métaux de transition et de métaux lourds. Par contre, les alcalins et alcalino-terreux ne sont que faiblement complexés par ces tétramines cycliques. Cette complexation sélective des cations de métaux de transition et de métaux lourds peut être mise à profit dans des applications potentielles très nombreuses. On peut en particulier envisager d'utiliser les tétramines cycliques à titre de résines échangeuses d'ions utiles notamment à l'extraction, la séparation ou le dosage des cations sélectionnés lors de la formation du complexe.

Cependant, pour mettre à profit les qualités d'un ligand macrocyclique dans l'une de ces diverses applications, il est nécessaire de modifier la structure de base du ligand, soit en lui adjoignant un groupement lipophile, soit en le fixant sur un polymère. Malheureusement, l'introduction sur un cycle d'un groupement fonctionnel quelconque rend presque toujours nécessaire la reprise complète de la synthèse du ligand macrocyclique.

La présente invention concerne plus particulièrement parmi les ligands macrocycliques, les tétramines dont le représentant le plus connu est le tétraaza-1,4,8,11 cyclotétradécane ou cyclam, de formule :

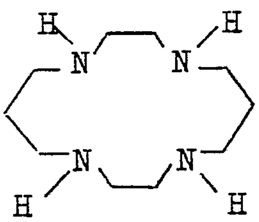

En ce qui concerne ces tétramines cycliques, une manière simple d'aborder le problème serait de fixer un groupement fonctionnel sur un des atomes d'azote du macrocycle par substitution nucléophile. En fait, une méthode générale de fonctionnalisation directe d'un seul des quatre atomes d'azote d'une tétramine cyclique n'a, jusqu'ici, jamais été décrite (2). La réactivité de ces molécules et les difficultés de séparation sont en effet telles que les auteurs préfèrent dans ce cas effectuer une nouvelle synthèse.

Au surplus, les rendements d'une telle synthèse sont très faibles. On arrive seulement à obtenir 10 à 20 % du produit voulu.

Ainsi, par exemple, pour réaliser la monoalkylation (3) d'une tétramine cyclique, on a dû procéder à diverses étapes réactionnelles schématisées de la manière suivante :

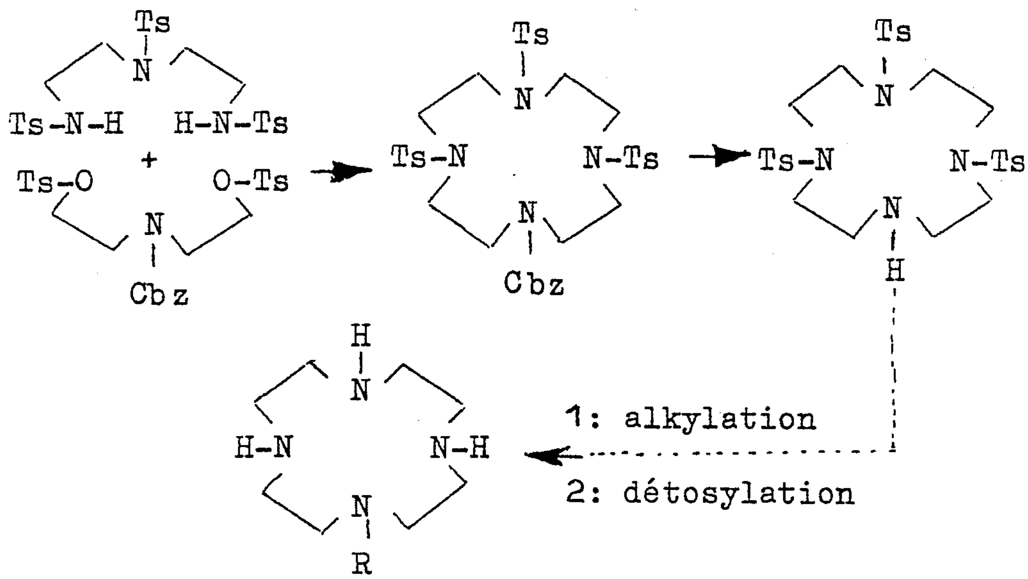


Dans ce schéma, Ts représente le groupe tosyle, Cbz représente le carbobenzyloxy et R représente le groupe alkyle avec lequel on désire fonctionnaliser le cycle.

La libération des N-tosyles constitue la dernière étape de cette synthèse. Une des méthodes les plus répandues consiste à faire réagir le dérivé tosylé dans $H_2SO_4$ concentré à 100°, pendant 24 à 48 heures. Ces conditions très vigoureuses limitent évidemment l'éventail des groupements R qu'il est possible d'introduire.

La présente invention permet de fonctionnaliser sur un atome d'azote les tétramines cycliques tout en évitant les inconvénients de la technique antérieure, tels que notamment la nécessité d'une nouvelle synthèse.

Le procédé selon la présente invention présente un intérêt industriel considérable :

les produits de départ sont des produits industriels et l'étape de fonctionnalisation, unique, est transposable à une plus grande échelle.

Le procédé selon l'invention comporte les étapes consistant à faire réagir un tétraazacycloalcane de formule II

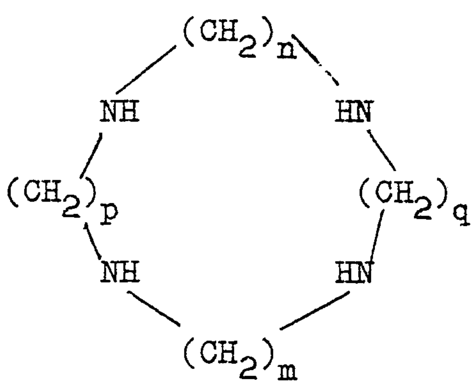

(II)

dans laquelle :

. n = m = p = q = 2 ou
. n = m = 2; p = q = 3 ou
. n = 2; m = p = q = 3 ou
. m = 2; n = p = q = 3 ou
. n = m = p = q = 3 ou
. n = m = 3; p = q = 4,

en quantité éventuellement excédentaire, avec un composé organique de formule :

X - R

dans laquelle : X représente un groupe nucléofuge, notamment un halogène ou un tosylate, et R un radical organique saturé ou non, notamment polymérisable, ladite réaction étant conduite dans un solvant polaire, à une température supérieure à environ 100° C, et en ce que, après refroidissement, on filtre le milieu réactionnel puis l'on procède à un lavage à l'aide d'une solution faiblement alcaline et à une extraction par un solvant organique.

On obtient ainsi une tétramine cyclique monofonctionnalisée de formule I suivante :

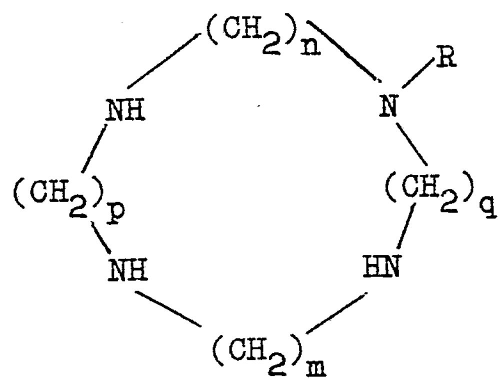

(I)

dans laquelle n, m, p, q et R ont les significations données ci-dessus.

Les tétramines cycliques les plus courantes, disponibles facilement dans le commerce, et que l'on utilisera donc de préférence dans le procédé de la présente invention, sont le cyclam, le tétraaza-1,4,7,10 cyclododécane répondant à la formule I ci-dessus dans laquelle n = m = p = q = 2, le tétraaza-1,4,8,12 cyclopentadécane répondant à la formule I ci-dessus, dans laquelle n = m = p = 3 et q = 2.

Le solvant polaire utile au procédé selon la présente invention peut être le DMSO ou l'acétonitrile, mais de préférence selon la présente invention on utilisera le N,N-diméthylformamide ou DMF et ce, notamment dans le cas où la tétramine cyclique utilisée est le cyclam.

En effet, le cyclam possède la particularité d'être très faiblement soluble à température ordinaire dans le DMF et de présenter une solubilité importante au-dessus de 100°C. Par ailleurs, le DMF, solvant polaire constitue, un milieu très favorable pour les réactions de substitution nucléophile.

L'utilisation du DMF dans cette réaction, autorise la présence d'un excès de cyclam, ce qui favorise naturellement la monoalkylation, sans compliquer la séparation du produit brut. En effet, la tétramine en excès précipite à froid et peut donc être éliminée facilement par simple filtration pour être éventuellement recyclée. Les produits de réaction restent par contre en solution.

Le lavage avec une solution légèrement alcaline permet de se débarrasser de la tétramine non fonctionnalisée encore présente ainsi que des ions $H^+$ et autres anions.

Préalablement à ce lavage, on peut, éventuellement, distiller le solvant polaire et ce, sous pression réduite et le résidu sera alors soumis au lavage précédemment cité.

Après ce lavage, on procède à une extraction par un solvant organique.

Ce solvant organique utilisé dans le procédé selon l'invention peut être quelconque. On utilisera de préférence le dichlorométhane ou le chloroforme.

Le produit brut ainsi extrait est, de préfé rence selon l'invention, soumis à une opération ultérieure de purification.

Pour ce faire, on le dissout dans un solvant organique tel que l'éthanol puis on ajoute un acide tel que l'acide sulfurique, jusqu'à précipitation complète. Après filtration, le précipité est repris par de la soude aqueuse et le produit organique purifié est extrait à l'aide d'un solvant approprié tel que le chlorure de méthylène.

Ce procédé permet d'obtenir des rendements supérieurs à 60 % en produits de monoalkylation d'excellente pureté. Si nécessaire, la préparation d'un complexe métallique, notamment celui de $Cu^{2+}$, $Ni^{2+}$ ou $Zn^{2+}$, permet par recristallisation, extraction sélective ou chromatographie, d'isoler un complexe de très haute pureté. La libération du ligand de l'ion inclus se fait selon les méthodes classiques de destruction du complexe par l'ion cyanure ou l'ion $H^+$.

La présente invention concerne aussi les tétramines cycliques qui ont été fonctionnalisées par le procédé mentionné ci-dessus, à savoir les composés de formule I :

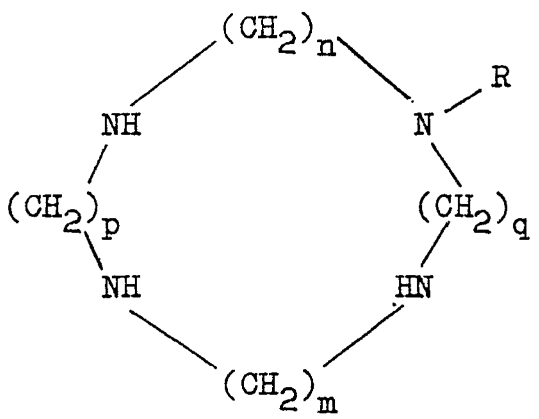

(I)

dans laquelle R représente un radical organique saturé ou non, notamment polymérisable, et
. n = m = p = q = 2 ou
. n = m = 2; p = q = 3 ou
. n = 2; m = p = q = 3 ou
. m = 2; n = p = q = 3 ou
. n = m = p = q = 3 ou
. n = m = 3; p = q = 4.

La présente invention concerne de préférence les composés de formule I dans laquelle : R représente

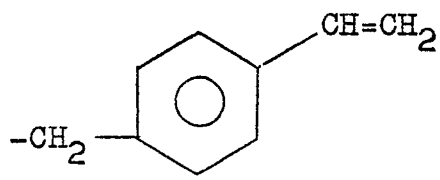

(forme para)

et/ou

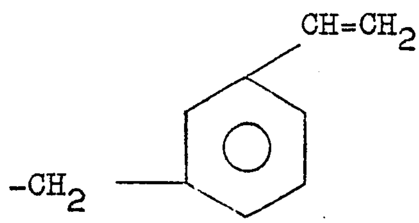

(forme méta).

Les composés tout particulièrement préférés dans le cadre de la présente invention sont les (vinyl benzyl)-1 tétraazacycloalcanes, répondant à la formule suivante :

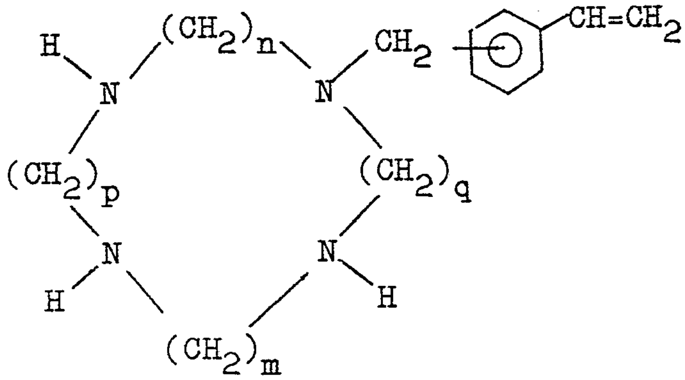

dans laquelle le radical -CH=$CH_2$ est placé en méta et/ou en para du noyau phényle.

Les produits de départ de tels composés sont les tétraazamacrocycles et le chlorométhylstyrène, disponibles dans le commerce. Le chlorométhylstyrène est fourni sous forme d'un mélange d'isomères (60 % méta et 40 % para, environ) dont la séparation a été récemment réalisée par voie chimique (4).

Le procédé de la présente invention a été mis en oeuvre à partir du mélange d'isomères et à partir de chacun des deux isomères purs, notamment l'isomère para pour le cyclam.

Compte tenu de la fragilité du groupement styrène, la synthèse de (vinyl benzyl)-1 tétraazacycloalcanes n'aurait jamais pu être obtenue par les procédés de la technique antérieure, sauf à recourir à des étapes de protection supplémentaires.

La présente invention s'étend aux polymères obtenus par homopolymérisation ou copolymérisation de l'une des tétramines cycliques fonctionnalisées selon le procédé décrit ci-dessus.

On peut effectuer une polymérisation radicalaire selon les procédés classiques. L'agent initiateur peut être l'AIBN, le peroxyde de benzoyle ou encore le rayonnement U.V. Si la purification a été convenablement réalisée, on obtient un polymère linéaire, soluble dans certains solvants tels que : eau, alcool, chloroforme, benzène, et insoluble dans d'autres solvants tels que le pentane et l'acétone.

La copolymérisation permettant d'obtenir un polymère réticulé peut être effectuée par des procédés connus de l'homme de l'art. En particulier, la copolymérisation avec un co-monomère tel que le divinylbenzène permet d'obtenir une résine réticulée insoluble dans l'eau et les solvants organiques. La polymé risation d'un monomère insuffisamment purifié conduit également à une résine insoluble, des traces de cyclam ou autre tétramine poly N-alkylée jouant alors le rôle d'agent réticulant.

Les résines ainsi obtenues présentent une très forte affinité pour les ions normalement complexés par le cyclam ou autre tétramine cyclique.

Par polymérisation des monomères selon l'invention, en présence d'agent réticulant, on obtient des résines échangeuses d'ions extrêmement sélectives, vis-à-vis des ions de transition et des métaux lourds. Ainsi $Cu^{2+}$, $Zn^{2+}$, $Cd^{2+}$, $Pb^{2+}$, $Mn^{2+}$, $Ni^{2+}$, $Co^{2+}$, $Ru^{3+}$, $Pd^{2+}$, etc. sont facilement retenus, même à l'état de traces.

Appliquées à la chimie de l'eau de mer (analyse des métaux-traces), ces résines sont plus performantes que celles classiquement utilisées (Chélex 100); notamment les étapes de préconcentration et d'élution sélective pour éliminer $Na^+$ ne sont pas nécessaires.

Le traitement des déchets radioactifs est une autre application intéressante, envisageable à une échelle industrielle.

En médecine, la purification du sang dans le cas des maladies comme la maladie de Wilson (empoisonnement du sang par $Cu^{2+}$) peut être envisagée grâce aux procédés et produits selon la présente invention.

La présente invention peut encore trouver d'autres applications nombreuses, notamment on peut incorporer les monomères ou polymères de la présente invention dans des produits de couchage en papeterie ou dans des peintures; ou encore leur utilisation comme électrolyte solide pour piles ou accumulateurs peut être envisagée; d'une manière générale, toutes les applications nécessitant une résine échangeuse d'ions de grande capacité (supérieure à 2,7 millimoles par gramme) peuvent être envisagées.

La présente invention est illustrée par les exemples suivants à l'aide des figures annexées qui représentent :
- fig. 1 : le pourcentage d'extraction des ions $Co^{2+}$, $Ni^{2+}$, $Cu^{2+}$, $Zn^{2+}$ en fonction du temps,
- fig. 2 : la cinétique de décomplexation de ces ions.
- fig. 3 : un schéma réactionnel illustrant l'exemple 15.

Parmi les exemples proposés, on notera le greffage de groupes lipophiles -$nC_{12}H_{25}$ ou $nC_{16}H_{33}$ ainsi que des groupements diversement fonctionnalisés. Les exemples n° 14 et n° 15 illustrent en outre la possibilité de transformation de ces groupements, notamment la possibilité de greffage sur un polymère du cyclam ainsi fonctionnalisé.

L'imprégnation des composés lipophiles sur un support inerte (polystyrène ou polyacrylate notamment) permet d'obtenir des résines échangeuses d'ions imprégnées telles que décrites dans la littérature (5). Ces composés lipophiles peuvent servir également à l'extraction ou au transfert de phase de cations et des anions

associés selon les méthodes décrites dans la littérature (6).

En incorporant un ion paramagnétique ou radioactif dans un ligand modifié selon notre méthode, les complexes ainsi obtenus pourraient trouver des applications en médecine, dans le domaine de l'imagerie RMN ou la scintigraphie.

D'une manière générale, notre méthode de monofonctionnalisation devrait permettre l'introduction facile d'un groupement nécessaire à l'application envisagée.

EXEMPLE 1 :

Préparation d'une tétramine fonctionnalisée de formule I dans laquelle R est un radical lipophile ($R = -n-C_{12}H_{25}$), n = m = 2 et p = q = 3 (rendement 65 %) :

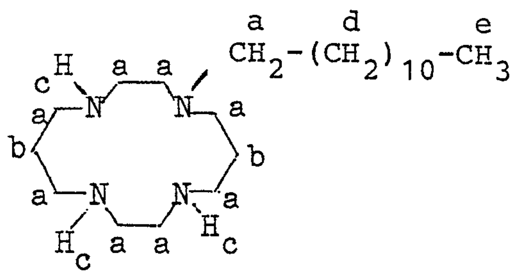

Les caractéristiques de ce produit sont les suivantes :

Aspect :

huile visqueuse claire

Pureté :

contrôlée par C.P.V.

conditions de séparation : colonne S.E. 30 1.40 m ;

gaz vecteur : $N_2$

température : four = 240°C, détecteur et injecteur = 270°C

Caractéristiques spectroscopiques :

- $^1H$ RMN : spectromètre JEOL FX 100 (100 MHz, solvant $CDCl_3$, référence : T.M.S.).
* 0,86 ppm (t, $3H_e$),
* 1,25 ppm (s, $20H_d$),
* 1,71 ppm (m, $4H_b$)
* 2,6 ppm (m, $3H_c$, 18 $H_a$)
- spectroscopie de masse : pic de masse $m/e^+ = 370$.

Application préférentielle :

Agent de transfert de phase.

EXEMPLE 2 :

Préparation d'une tétramine fonctionnalisée de formule I dans laquelle R est un radical nitrile ($R = -(CH_2)_4-C \equiv N$), n = m = 2 et p = q = 3 (rendement 65 %) :

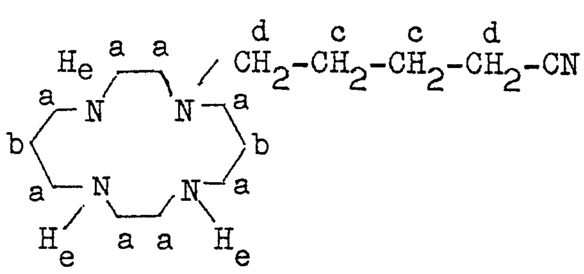

Ses caractéristiques sont les suivantes :

Aspect :

huile visqueuse claire

Caractéristiques spectroscopiques

- $^1H$ RMN : spectromètre JEOL FX 100 (100 MHz; solvant $CDCl_3$; référence : T.M.S.).

* 1,74 ppm (m, $4H_b$, $4H_c$)
* 2,62 ppm (m, $3H_e$, $16H_a$, $4H_d$)
-spectroscopie de masse : pic de masse $m/e^+ = 282$.
Application préférentielle
Modification de la fonction terminale $-C \equiv N$ pour un éventuel greffage sur polymère ou une réaction ultérieure.

Exemple 3 :
Préparation d'une tétramine fonctionnalisée de formule I dans laquelle $R = -CH_2-(C_6H_4)-CH=CH_2$ (mélange d'isomères méta-para),
$n = m = 2$ et $p = q = 3$ :

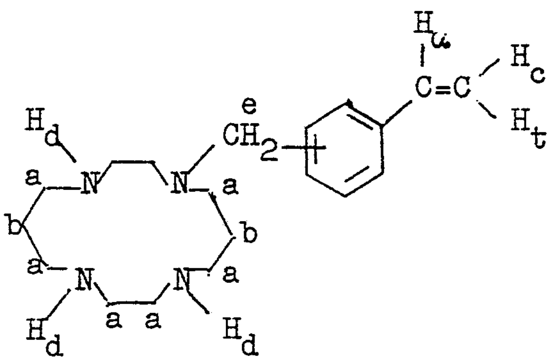

a) Conditions opératoires
Un excès de cyclam favorise la monoalkylation. On rapporte ici les résultats obtenus avec un rapport cyclam/chlorométhylstyrène proche d'environ cinq.
Le cyclam (40 g) est versé dans 1 litre de DMF sec et la solution est portée aux environs de 110°C. On ajoute ensuite lentement 3 g de chlorométhylstyrène fraîchement distillé. On laisse réagir une heure. Après refroidissement du mélange, filtration et traitement comme indiqué précédemment, on obtient 3,75 g de produit de monoalkylation (rendement environ 60 %).

b) Caractéristiques du produit obtenu Aspect :
huile visqueuse claire
Pureté :
contrôlée par C.P.V.
condition de séparation : colonne S.E. 30 1,40 m;
gaz vecteur $N_2$
température : four 220°C, détecteur et injecteur = 270°C.
Caractéristiques spectroscopiques
- $^1H$ RMN : spectromètre JEOL FX 100 (100 MHz, solvant $CDCl_3$; référence TMS)
*1,85 ppm (m, $4H_b$),
* 2,70 ppm (m, $16H_a$, $3H_d$)
* 3,55 ppm (s, $2H_e$)
* 5,15 ppm (dd, $J_{cis} = 11$ Hz, $J_{gem} = 0,7$Hz, $1H_c$)
* 5,75 ppm (dd, $J_{trans} = 17,6$ Hz, $J_{gem} = 0,7$ Hz, $1H_t$)
* 6,70 ppm (dd, $J_{trans} = 17,6$ Hz, $J_{cis} = 11$Hz, $1H_{\alpha}$)
* 7,25 ppm (m, $2H_{\emptyset}$)
- spectroscopie de masse : pic de masse $m/e^+ = 316$

c) Polymères linéaire et réticulé de ce produit
Les conditions d'obtention, l'aspect et la transition vitreuse, la capacité d'absorption des ions métalliques sont comparables à l'isomère para pur (voir exemple 4).
Les cinétiques de complexation et de décomplexation du polymère réticulé sont représentées sur les figures 1 et 2 avec les conditions d'extraction suivantes :
. 20 ml de solution $1,6.10^{-2}$M (sulfate) 50 mg de polymère sec (cyclam sur polymère : mélange d'isomères méta-para), granulométrie 75-150 microns; ion/ligand $\simeq 2$.
La cinétique de décomplexation par HCl 6N est référencée sur la figure 2. Application préférentielle
Résine échangeuse d'ions.

EXEMPLE 4 :

Préparation d'une tétramine fonctionnalisée de formule I dans laquelle R = $-CH_2-(C_6H_4)-CH = CH_2$ (isomère para), n = m = 2 et p = q = 3 (rendement 65 %) :

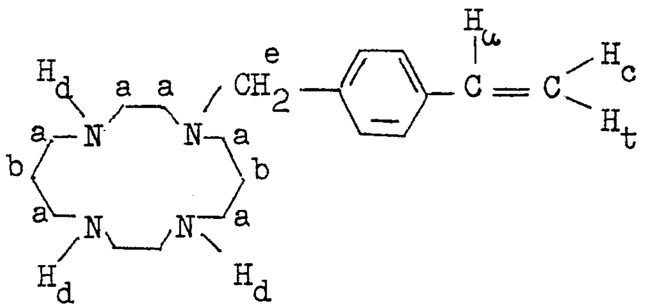

a) Caractéristiques de ce produit Aspect :

huile visqueuse claire

Pureté :

contrôlée par C.P.V.

conditions de séparation : colonne S.E. 30 1,40 m;

gaz vecteur $N_2$

températures : four = 220°C, détecteur et injecteur = 270°C.

Caractéristiques spectroscopiques

- $^1H$ RMN : spectromètre JEOL FX 100 (100 MHz; solvant $CDCl_3$ référence T.M.S.)

* 1,73 ppm (m, $4H_b$)
* 2,85 ppm (m, $16H_a$, $3H_d$),
* 3,55 ppm (s, $2H_e$)
* 5,18 ppm (dd, $J_{cis}$ = 11Hz $J_{gem}$ = 0,7 Hz, $1H_c$)
* 5,68 ppm (dd, $J_{trans}$ = 17,6 Hz, $J_{gem}$ = 0,7 Hz, $1H_t$)
* 6,66 ppm (dd, $J_{trans}$ = 17,6 Hz, $J_{cis}$ = 11Hz, $1H\alpha$)
* 7,25 ppm (m, 4H ø)

- spectroscopie de masse : pic de masse $m/e^+$ = 316

b) Polymérisation de ce produit

b.1. Polymère linéaire

Conditions d'obtention : au monomère en solution dans le benzène, on ajoute de l'AIBN comme agent d'initiation. La réaction s'effectue à 60-65°C sous atmosphère d'azote.

Aspect :

solide blanc, soluble entre autres dans l'eau, le benzène, l'éthanol, le méthanol, le dichlorométhane, le chloroforme, insoluble dans l'acétone et l'éther de pétrole.

Transition vitreuse vers 95°C.

b.2. Polymère réticulé

Condition d'obtention : au monomère en solution dans le benzène, on ajoute de l'AIBN comme agent d'initiation, et du divinylbenzène, comme agent réticulant. La réaction s'effectue à 60-65°C sous atmosphère d'azote.

Aspect :

solide jaune pâle insoluble dans l'eau et les solvants organiques

Transition vitreuse vers 95°C.

Capacité d'absorption des ions métalliques.

1) Calculée : 3,16 mmoles par gramme de résine anhydre

2) Mesurée : sur 100 mg de résine sèche de granulométrie 75-150 microns = 2,98 mmoles par gramme de résine pour les ions $Cu^{2+}$, $Zn^{2+}$, $Co^{2+}$ et $Ni^{2+}$.

3) Cinétique de complexation rapide (voir figure jointe) conditions : 20 ml de solution $1,6.10^{-2}$ (sulfate), 50 mg de polymère sec, granulométrie 75-150 microns, ion/ligand ≃2

Application préférentielle

Résine échangeuse d'ions.

EXEMPLE 5 :

Préparation du mélange des deux tétramines fonctionnalisées suivantes (rendement 80 %) :

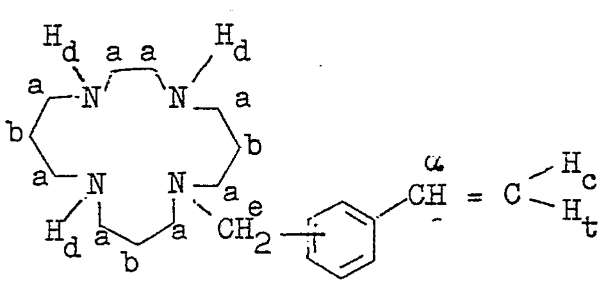

+

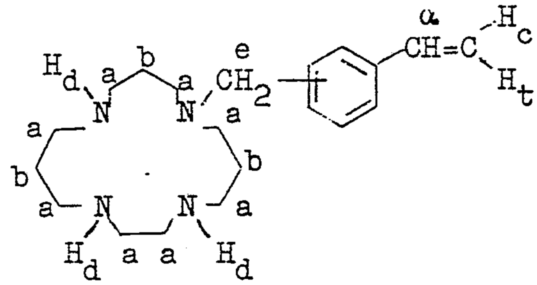

a) Caractéristiques du produit obtenu Aspect :

huile visqueuse claire

Caractéristiques spectroscopiques

- $^1H$ RMN : spectromètre JEOL FX 100 (100 MHz, solvant $CDCl_3$, référence TMS).
* 1,66 ppm (m, $6H_b$)
* 2,58 ppm (m, $16H_a$, $3H_d$)
* 3,48 ppm et 3,49 ppm (les 2 singulets des $2H_e$ des deux isomères)
* 5,23 ppm (dd, $J_{cis}$ = 11Hz, $J_{gem}$ = 0,7Hz, $1H_c$)
* 5,82 ppm (dd, $J_{trans}$ = 17,6Hz, $J_{gem}$ = 0,7Hz, $1H_t$)
* 6,71 ppm (dd, $J_{trans}$ = 17,6Hz, $J_{cis}$ = 11 Hz, $1H_\alpha$)
* 7,27 ppm (m, $4H_\emptyset$)
- Spectroscopie de masse : pic de masse $m/e^+ = 330$

b) Polymère réticulé

Les conditions d'obtention et l'aspect sont comparables à ceux de l'isomère para pur du dérivé obtenu avec le cyclam comme tétramine.

Transition vitreuse vers 100°C

Capacité d'absorption des ions métalliques

a - calculée : 3,03 mmoles par gramme de résine anhydre

b - mesurée : sur 100 mg de résine sèche de granulométrie 75-150 microns : 2,79 mmoles par gramme de résine pour les ions $Cu^{2+}$, $Zn^{2+}$, $Ni^{2+}$ et $Co^{2+}$.

c - cinétique de complexation rapide : plus de 95 % des sites sont saturés en moins de cinq minutes par les ions $Cu^{2+}$, $Zn^{2+}$, $Ni^{2+}$ et $Co^{2+}$. Conditions 20 ml de solution $1{,}5.10^{-2}$ (sulfate), 50 mg de polymère sec, granulométrie 75-150 microns, ion/ligand $\simeq 2$.

d - la cinétique de décomplexation par HCl 6N est représentée sur la figure 2. Application préférentielle

Résine échangeuse d'ions

EXEMPLE 6

Préparation d'une tétramine fonctionnalisée de formule I dans laquelle R est un radical ($R = -(CH_2)_5-CH_2OH$), n = m = 2 et p = q = 3 (Rendement $\simeq$ 65 %)

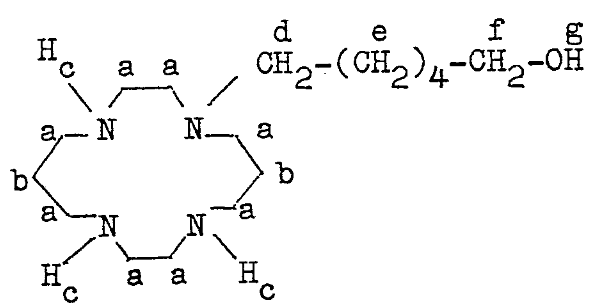

Les caractéristiques de ce produit sont les suivantes :

Aspect :

huile visqueuse claire

Caractéristiques spectroscopiques :

- $^1H$ RMN : spectromètre JEOL FX 100 (100 MHz, solvant $CDCl_3$, Référence : T.M.S.)
* 1,54 ppm (m, 4 $H_b$, 8 $H_e$)
* 2,61 ppm (m, 16 $H_a$, 2 $H_d$)
* 3,50 ppm (m, 1 $H_g$, 3 $H_c$)
* 3,58 ppm (t, 2 $H_f$)
- Spectroscopie de masse : pic de masse $m/e^+ = 301$.

Application préférentielle

Possibilité de modifier la fonction terminale en vue d'une réaction ou d'un greffage ultérieurs.

EXEMPLE 7

Préparation d'une tétramine fonctionnalisée de formule I dans laquelle $R = -CH_2-(C_6H_4)-CH=CH_2$ (mélange d'isomères méta-para) m = n = p = q = 2 (Rendement ≃ 60 %)

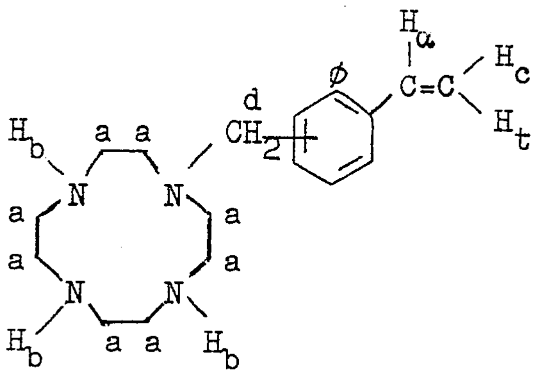

a) Conditions opératoires

2,5 g de cyclen est versé dans 50 ml d'acétonitrile et la solution est portée à reflux. On ajoute ensuite lentement 445 mg de chlorométhylstyrène fraîchement distillé (rapport cyclen/chlorométhylstyrène proche d'environ cinq). On laisse réagir deux heures. Après refroidissement du mélange, filtration et traitement comme indiqué précédemment, on obtient 505 mg de produit de monoalkylation (rendement environ 60 %).

b) Caractéristiques du produit obtenu Aspect :

huile visqueuse claire

Caractéristiques spectroscopiques

- $^1H$ RMN : spectromètre JEOL FX 100 (100 MHz, solvant $CDCl_3$, Référence T.M.S.)
* 2,67 ppm (m, 16 $H_a$, 3 $H_b$)
* 3,59 ppm (s, 2 $H_d$ des isomères méta et para)
* 5,19 ppm (dd, $J_{cis} = 11$ Hz, $J_{gem} = 0,7$ Hz, 1 $H_c$)
* 5,71 ppm (dd, $J_{trans} = 17,6$ Hz, $J_{gem} = 0,7$ Hz, 1 $H_t$)
* 6,67 ppm (dd, $J_{trans} = 17,6$ Hz, $J_{cis} = 11$ Hz, 1 $H_\alpha$)
* 7,27 ppm (m, 4 $H_\phi$)
- Spectroscopie de masse = pic de masse $m/e^+ = 289$.

c) Polymères linéaires et réticulés de ce produit

c.1. Polymère linéaire

Conditions d'obtention : comparable à l'isomère para pur du cyclam (cf. exemple 4).

Aspect :

solide blanc, soluble entre autres dans l'eau, le benzène, l'éthanol, le méthanol, le dichlorométhane, le chloroforme, insoluble dans l'acétone et l'éther de pétrole.

Transition vitreuse vers 60-65°C.

c.2. Polymère réticulé

Condition d'obtention : comparable à l'isomère para pur du cyclam (cf. exemple 4).

Aspect :

solide blanc insoluble dans l'eau et les solvants organiques.

Complexation et décomplexation des ions métalliques. La cinétique de complexation des ions métalliques ($Cu^{2+}$, $Zn^{2+}$, $Co^{2+}$, $Ni^{2+}$) est rapide (voir figure I) (Capacité 3,47 mmoles par gramme de résine anhydre). Conditions : 20 ml de solution 1,7 $10^{-2}$ (sulfate), 30 mg de polymère sec, ion/ligand $\simeq$ 2.

Cinétique de décomplexation (cf. figure 2). La désorption est complète en moins d'une heure lorsque le polymère saturé par les ions $Cu^{2+}$, $Zn^{2+}$ et $Ni^{2+}$ est traité par HCl 6N. Dans ces conditions, et même avec HCl 11N, le cobalt n'est pas décomplexé. Le cobalt est facilement décomplexé par traitement avec une solution de KCN 2M à 60°C. Application préférentielle :

Résine échangeuse d'ions.

EXEMPLE 8

Préparation d'une tétramine fonctionnalisée de formule I dans laquelle R = $-n(C_{16}H_{33})$, n = m = p = q = 2 (rendement $\simeq$ 60 %).

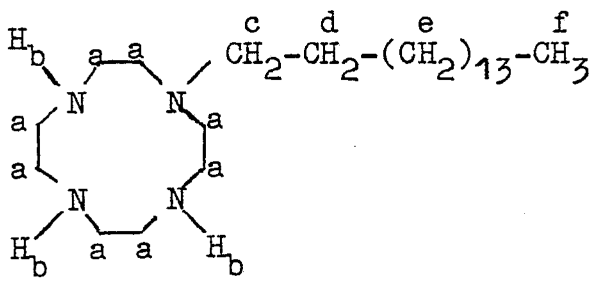

Les caractéristiques de ce produit sont les suivantes :

Aspect :

huile visqueuse claire

Caractéristiques spectroscopiques

. $^1H$ RMN : spectromètre JEOL FX 100 (100 MHz, solvant $CDCl_3$, Référence T.M.S.)

* 0,87 ppm (t, 3 $H_f$)
* 1,24 ppm (s, 26 $H_e$)
* 1,44 ppm (m, 2 $H_d$)
* 2,57 ppm (m, 16 $H_a$, 3 $H_b$, 2 $H_c$)

- Spectroscopie de masse = pic de masse $m/e^+$ = 397. Application préférentielle

Agent de transfert de phase.

EXEMPLE 9

Préparation d'une tétramine fontionnalisée de formule I dans laquelle

R = 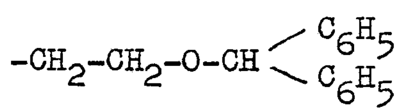 ,

n = m = p = q = 2 (Rendement $\simeq$ 70 %)

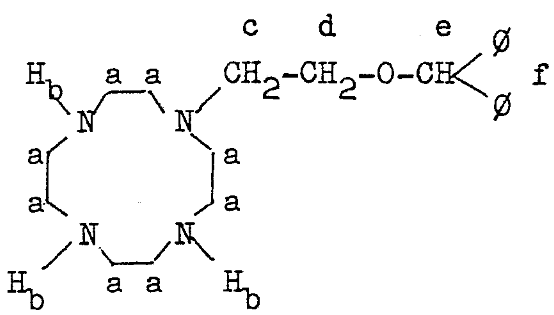

Les caractéristiques de ce produit sont les suivantes :

Aspect :

huile visqueuse claire

Caractéristiques spectroscopiques

. $^1H$ RMN : spectromètre JEOL FX 100 (100 MHz, solvant $CDCl_3$, référence T.M.S.

* 2,60 ppm (m, 16 $H_a$, 3 $H_b$)
* 2,73 ppm (t, 2 $H_c$)
* 3,56 ppm (t, 2 $H_d$)
* 5,40 ppm (s, 1 $H_e$)
* 7,28 ppm (m, 10 $H_f$)

. Spectroscopie de masse = pic de masse $m/e^+$ = 383 Applications préférentielles

Médecine nucléaire et transfert de phase.

EXEMPLE 10

Préparation d'une tétramine fonctionnalisée de formule I dans laquelle R = $-CH_2-(C_6H_4)-CH=CH_2$ (mélange d'isomère méta-para) n = m = p = q = 3 (Rendement ≃ 60 %)

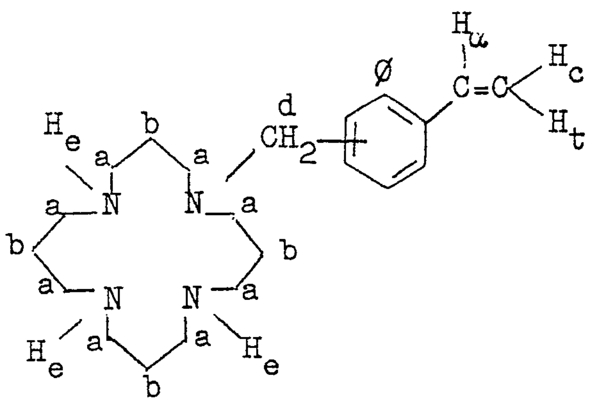

a) Conditions opératoires

2,5 g de tétraaza-1,5,9,13 cyclohexadécane ([16] ane $N_4$) sont versés dans 75 ml d'acétonitrile et la solution est portée à reflux. On ajoute ensuite 335 mg de chlorométhylstyrène fraîchement distillé (rapport [16] ane $N_4$/chlorométhylstyrène proche d'environ cinq). On laisse réagir deux heures. Après refroidissement du mélange, filtration et traitement comme indiqué précédemment, on obtient 450 mg de produit de monoalkylation (rendement environ 60 %).

b) Caractéristiques du produit obtenu Aspect :

huile visqueuse claire

Caractéristiques spectroscopiques

- $^1H$ RMN : spectromètre JEOL FX 100 (100 MHz, solvant $CDCl_3$, référence T.M.S.)

* 1,74 ppm (m, 8 $H_b$)
* 2,58 ppm (m, 16 $H_a$, 3 $H_e$)
* 3,51 ppm (s, 2 $H_d$ des isomères méta et para)
* 5,23 ppm (dd, $J_{cis}$ = 11 $H_z$, $J_{gem}$ = 0,7 $H_z$, 1 $H_c$)
* 5,74 ppm (dd, $J_{trans}$ = 17,6 Hz, $J_{gem}$ = 0,7 Hz, 1 $H_t$)
* 6,71 ppm (dd, $J_{trans}$ = 17,6 Hz, $J_{cis}$ = 11 Hz, 1 $H_\alpha$)
* 7,27 ppm (m, 4 $H_\emptyset$)

- Spectroscopie de masse = pic de masse $m/e^+$ = 345.

Application préférentielle

Résine échangeuse d'ions.

EXEMPLE 11

Préparation d'une tétramine fonctionnalisée de formule I dans laquelle R = $-nC_{12}H_{25}$, n = m = p = q = 3 (rendement ≃ 60 %)

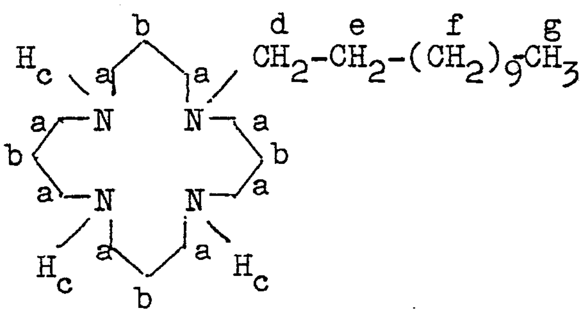

Les caractéristiques de ce produit sont les suivantes :

Aspect :

huile visqueuse claire

Caractéristiques spectroscopiques

- $^1H$ RMN : spectromètre JEOL FX 100 (100 MHz, solvant $CDCl_3$, référence T.M.S.)
* 0,88 ppm (t, 3 $H_g$)
* 1,26 ppm (s, 18 $H_f$)
* 1,41 ppm (m, 2 $H_e$)
* 1,64 ppm (m, 8 $H_b$)
* 2,56 ppm (m, 16 $H_a$, 3 $H_c$, 2 $H_d$)
- Spectroscopie de masse = pic de masse $m/e^+ = 397$

Application préférentielle

Agent de transfert de phase.

EXEMPLE 12

Préparation d'une tétramine fonctionnalisée de formule I dans laquelle R = $-nC_{16}H_{33}$, n = m = 3 et p = q = 4 (Rendement ≃ 60 %)

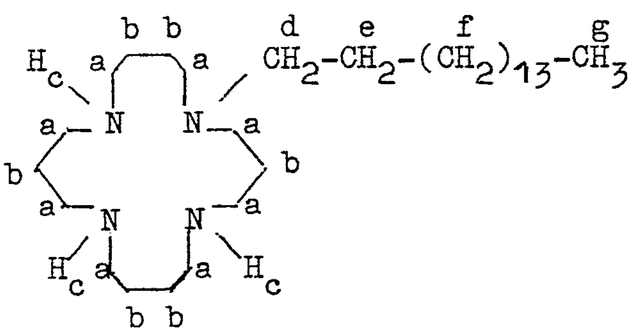

a) Conditions opératoires cf. exemple 10.

b) Caractéristiques du produit obtenu Aspect :

huile visqueuse claire

Caractéristiques spectroscopiques

- $^1H$ RMN : spectromètre JEOL FX 100 (100 MHz, solvant $CDCl_3$, référence T.M.S.)
* 0,86 ppm (t, 3 $H_g$)
* 1,28 ppm (s, 26 $H_f$)
* 1,40 ppm (m, 2 $H_e$)
* 1,60 ppm (m, 12 $H_b$)
* 2,67 ppm (m, 16 $H_a$, 3 $H_c$, 2 $H_d$)
- Spectroscopie de masse = pic de masse $m/e^+ = 524$.

Application préférentielle :

Agent de transfert de phase.

EXEMPLE 13

Préparation d'une tétramine fonctionnalisée de formule I dans laquelle R = -n-$(CH_2)_5$-$CH_2OH$, n = m = 3 et p = q = 4 (Rendement ≃ 65 %)

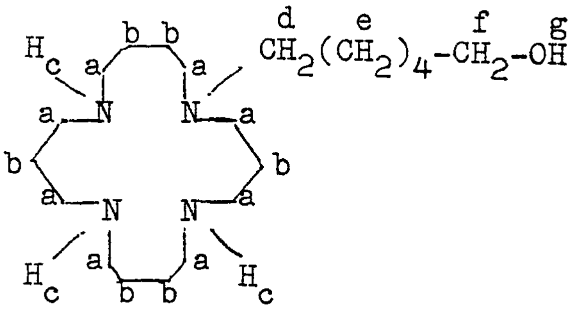

Les caractéristiques de ce produit sont les suivantes :

Aspect :

huile visqueuse claire

Caractéristiques spectroscopiques

. $^1H$ RMN : spectromètre JEOL FX 100 (100 MHz, solvant $CDCl_3$, référence T.M.S.)

* 1,49 ppm (m, 12 $H_b$, 8 $H_e$)
* 2,60 ppm (m, 16 $H_a$, 3 $H_c$, 2 $H_d$, 1 $H_g$)
* 3,58 ppm (t, 2 $H_f$)

. Spectroscopie de masse = pic de masse m/$e^+$ = 357

Application préférentielle

Modification possible de la fonction terminale.

EXEMPLE 14

Préparation d'une tétramine fonctionnalisée de formule I dans laquelle R = -$(CH_2)_5$-$NH_2$, n = m = 2 et p = q = 3.

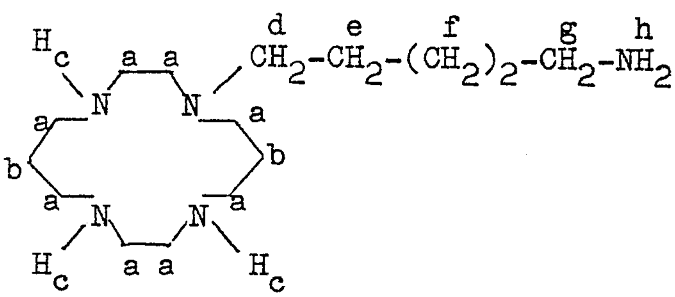

a) Conditions opératoires

A 332 mg d'hydrure de lithium aluminium dans 40 ml d'éther éthylique anhydre, on ajoute goutte à goutte 1,230 g de cyclam monofonctionnalisé par un radical nitrile (exemple 2) en solution dans l'éther éthylique anhydre. On laisse réagir 45 minutes à reflux. Le ballon est refroidi. On hydrolyse avec de la soude à 5 % jusqu'à obtention d'un précipité blanc. Le milieu réactionnel est alors séché avec du sulfate de magnésium et filtré. On évapore l'éther sous pression réduite. On obtient 1,13 g d'amine pure avec un rendement de l'ordre de 90 %.

b) Caractéristiques du produit obtenu - $^1H$ RMN : spectromètre JEOL FX 100 (100 MHz, solvant $CDCl_3$, référence T.M.S.)

* 1,56 ppm (m, 4 $H_b$, 2 $H_e$, 4 $H_f$)
* 2,50 ppm (m, 16 $H_a$, 3 $H_c$, 2 $H_d$), 2 $H_g$, 2 $H_h$)

- Spectrométrie de masse = pic de masse m/$e^+$ = 286

Applications préférentielles

(exemple de modification de la fonction terminale du composé de l'exemple 2).
Fonction modifiable pour une réaction ou un greffage ultérieurs et résine échangeuse d'ions.

EXEMPLE 15

Préparation d'une tétramine fonctionnalisée de formule I greffée sur polymère.

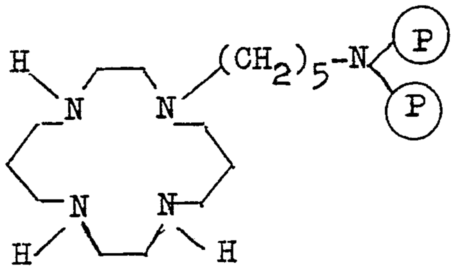

a) Schéma réactionnel :

voir figure 3.

b) Conditions opératoires

A 3,45 g d'amine dans 500 ml de DMF on ajoute 2,1 g de $CuCl_2$, 2 $H_2O$. On laisse réagir 30 minutes. Le blocage des quatre azotes du cycle est ainsi assuré par l'ion $Cu^{2+}$. On porte la température à 100-110°C et on ajoute 920 mg de polychlorométhylstyrène en solution dans le DMF (mélange d'isomère orthopara). Dans ces conditions, l'azote terminal réagit deux fois pour donner à ce niveau une amine tertiaire. On laisse réagir pendant 24 heures. Le DMF est évaporé partiellement sous pression réduite, puis on verse le mélange réactionnel dans 500 ml d'eau. Par filtration, on obtient le polymère qui est lavé trois fois avec une solution d'acide chlorhydrique 6N à chaud, une fois à l'eau, à l'ammoniaque puis encore une fois à l'eau. Après séchage, on obtient 1 g de polymère (R = 65 %). Dans ces conditions, le cyclam ne réagit pas.

c) Cinétique de complexation et de décomplexation :

voir figures 1 et 2. Application préférentielle

(Exemple de modification de la fonction terminale de composé de l'exemple 14).

Résine échangeuse d'ions.

BIBLIOGRAPHIE

(1) a - Host Guest Complex Chemistry I, II, III, F. VOGTLE et E. WEBER Edit., SPRINGER Verlag, 1980, 1981 et 1984.

b - Coordination Chemistry of Macrocyclic Compounds. A. MELSON, Edit., PLENUM, 1979.

(2) T.A. KADEN dans Host Guest Complex Chemistry III, F. VOGTLE et E. WEBER Edit., SPRINGER Verlag, 1984, page 162.

(3) J.F. PILICHOWSKI, Thèse de Doctorat d'Etat, Clermont-Ferrand, 1983.

(4) M. CAMPS. M. CHATZOPOULOS, J.P. MONTHEARD et Q.T. PHAM, Makromol Chem., Rapid Commun., 1982, 3, 35.

(5) F. MULLER, H. HANDEL et R. GUGLIELMETTI, Helv. Chim. Acta., 1983,66,1525.

(6) H. HANDEL, F. MULLER et R. GUGLIELMETTI, Helv. Chim. Acta., 1983,66,514.

**Revendications**

1 - Procédé de préparation de tétramines cycliques monofonctionnalisées de formule I suivante :

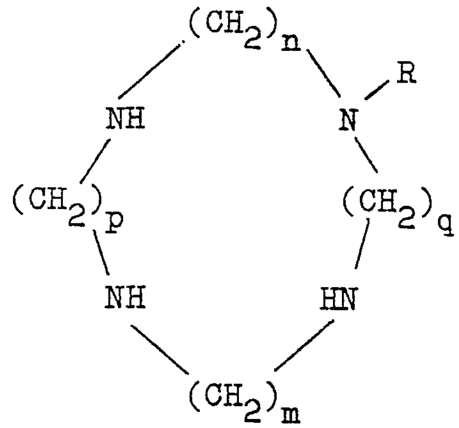

(I)

dans laquelle :
n = m = p = q = 2 ou
n = m = 2; p = q = 3 ou
n = 2; m = p = q = 3 ou
m = 2; n = p = q = 3 ou
n = m = p = q = 3 ou
n = m = 3; p = q = 4,

R représente un radical organique saturé ou non, notamment polymérisable, caractérisé en ce que l'on fait réagir un tétraazacycloalcane de formule II

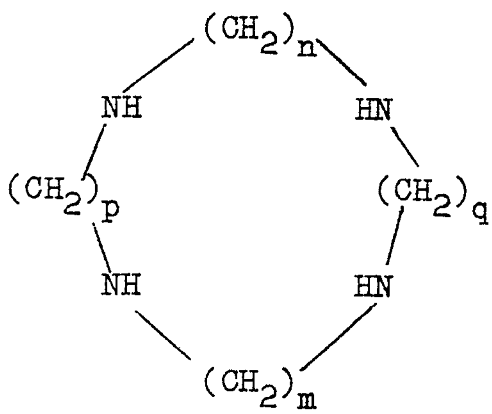

(II)

dans laquelle n, m, p et q ont les significations données ci-dessus, en quantité éventuellement excédentaire, avec un composé organique de formule :

X - R

dans laquelle X représente un groupe nucléofuge, notamment un halogène ou un tosylate, et R un radical organique saturé ou non, notamment polymérisable, ladite réaction étant conduite dans un solvant polaire à une température supérieure à environ 100° C, et en ce que, après refroidissement, on filtre le milieu réactionnel puis l'on procède à un lavage à l'aide d'une solution faiblement alcaline et à une extraction par un solvant organique.

2 - Procédé selon la revendication 1, caractérisé en ce que le solvant polaire est choisi parmi le DMSO, l'acétonitrile et le DMF.

3 - Procédé selon la revendication 1 ou 2, caractérisé en ce que le solvant organique est le dichlorométhane ou le chloroforme.

4 - Procédé selon l'une quelconque des revendications 1 à 3, caractérisé en ce que le produit brut ainsi obtenu est soumis à une opération ultérieure de purification par dissolution dans un solvant organique, notamment l'éthanol, puis addition d'un acide, en particulier l'acide sulfurique, jusqu'à précipitation complète, le précipité ainsi obtenu étant repris par de la soude aqueuse et le produit organique isolé par extraction à l'aide d'un solvant approprié tel que le chlorure de méthylène.

5 - Tétramines cycliques monofonctionnalisées de formule I

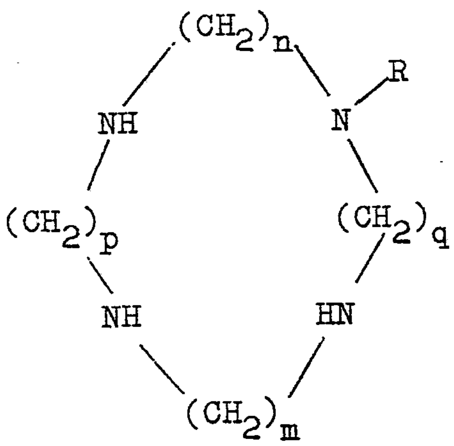

(I)

dans laquelle R représente un radical organique saturé ou non, notamment polymérisable, et
n = m = p = q = 2 ou
n = m = 2 ; p = q = 3 ou
n = 2 ; m = p = q = 3 ou
m = 2 ; n = p = q = 3 ou
n = m = p = q = 3 ou
n = m = 3 ; p = q = 4,

obtenus par le procédé selon l'une quelconque des revendications 1 à 5.

6 - Tétramines cycliques monofonctionnalisées selon la revendication 5, caractérisée en ce qu'elles répondent à la formule suivante :

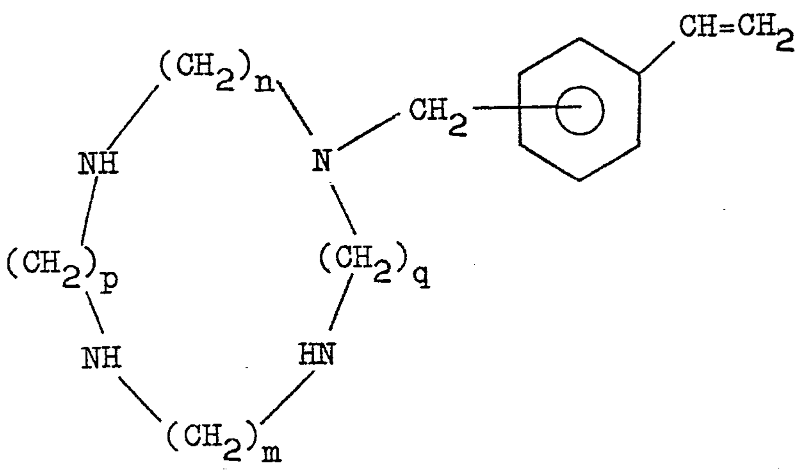

dans laquelle :
n = m = p = q = 2 ou
n = m = 2 ; p = q = 3 ou
n = 2 ; m = p = q = 3 ou
m = 2 ; n = p = q = 3 ou
n = m = p = q = 3 ou
n = m = 3 ; p = q = 4,

et le dérivé $-CH=CH_2$ est placé en méta et/ou en para du noyau phényle.

7 - Polymères linéaires obtenus par homopolymérisation de l'une des tétramines selon l'une quelconque des revendications 5 ou 6.

8 - Polymères réticulés obtenus par copolymérisation de l'une des tétramines selon l'une quelconque des revendications 5 ou 6 avec un co-monomère tel que le divinylbenzène.

9 - Utilisation des polymères selon l'une quelconque des revendications 7 ou 8, au titre de résine échangeuse d'ions.

10 - Utilisation des polymères selon la revendication 9 pour l'extraction sélective et/ou le dosage des ions des métaux de transition et des métaux lourds.

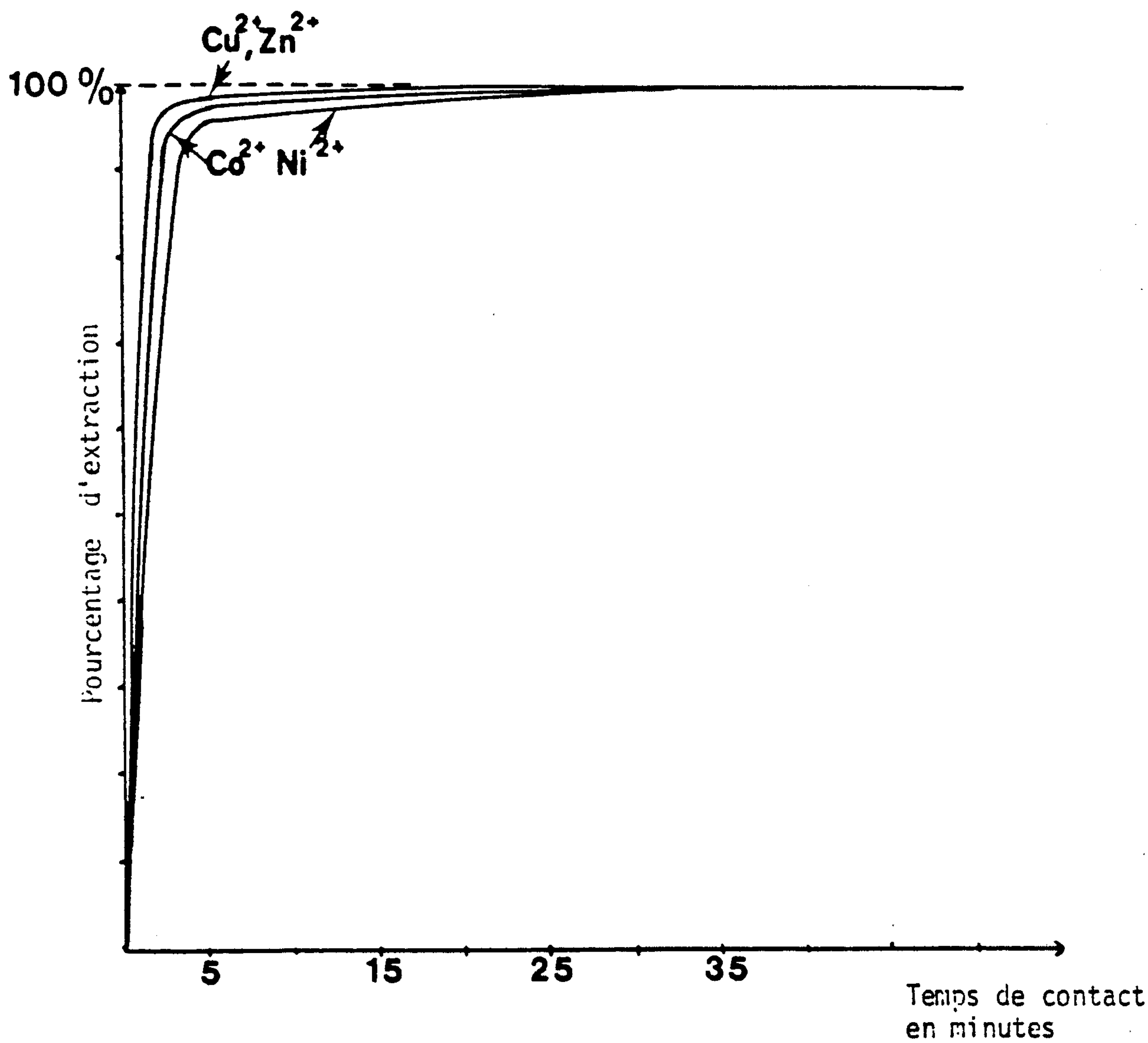
Cu2+, Zn2+
100 %
Co2+ Ni2+
Pourcentage d'extraction
5
15
25
35
Temps de contact
en minutes

FIGURE 1

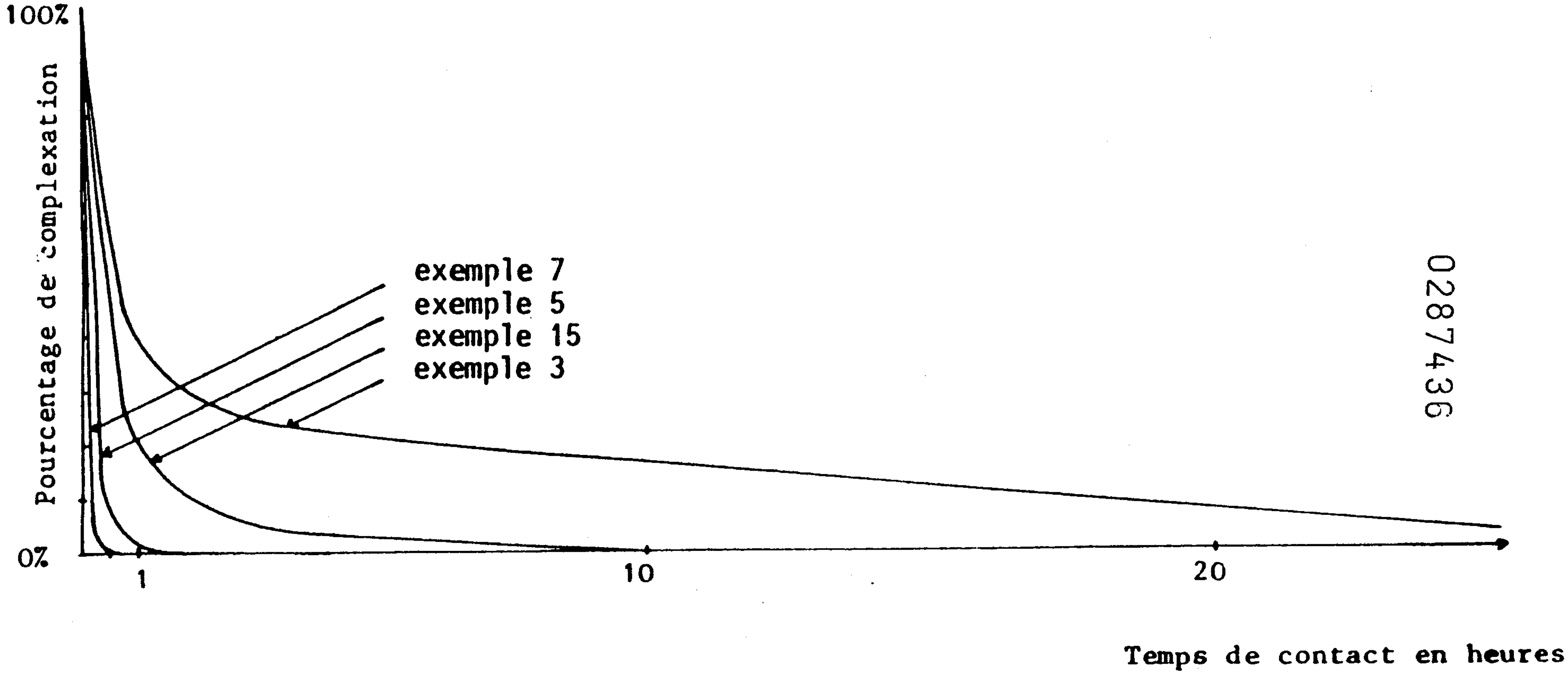
100%
0%
Pourcentage de complexation
1
10
20
Temps de contact en heures
exemple 7
exemple 5
exemple 15
exemple 3

Figure 2

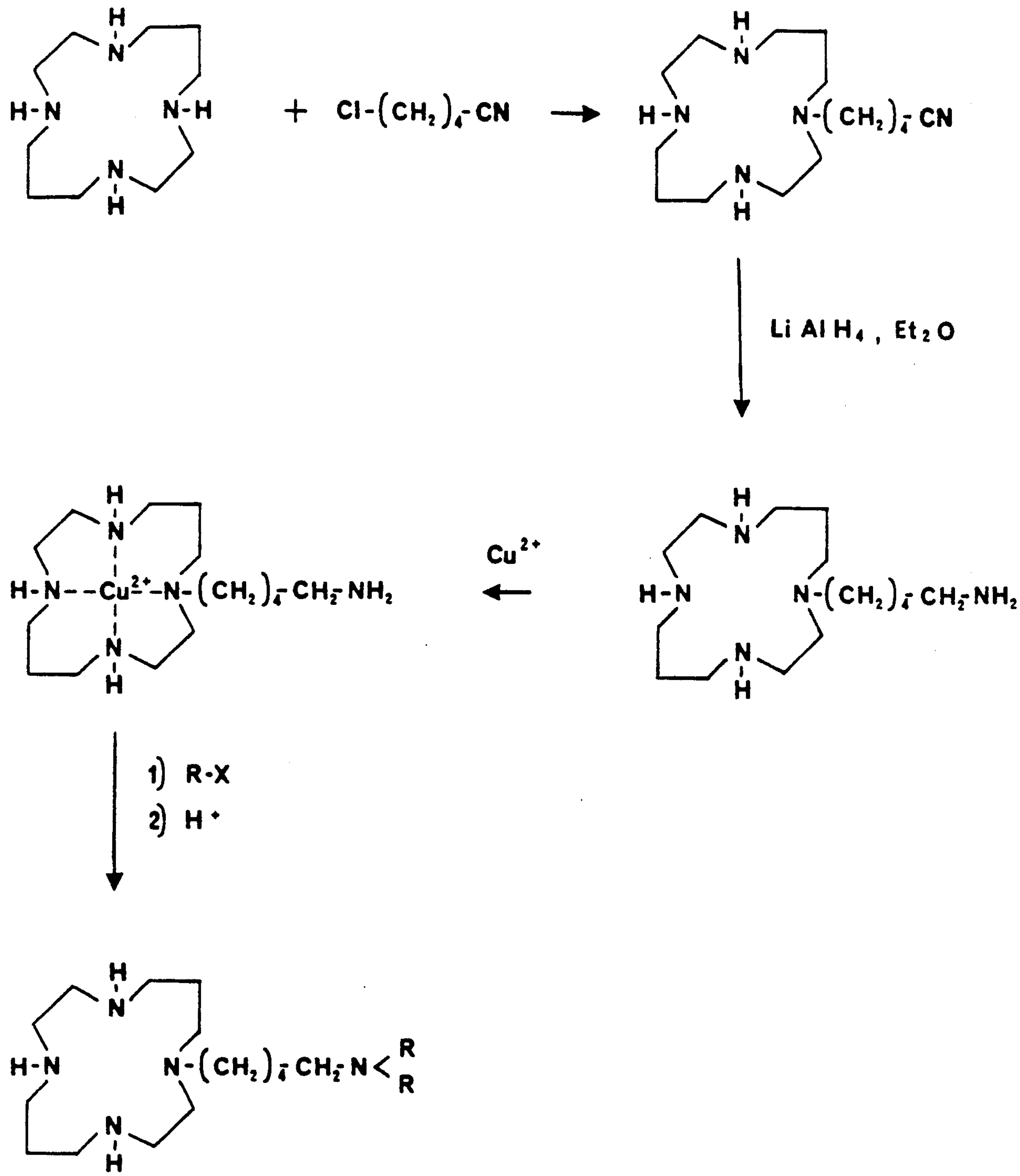


Figure 3

Office européen des brevets

# RAPPORT DE RECHERCHE EUROPEENNE

Numero de la demande

EP 88 40 0839

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A-1 529 150 (I.I. TABUSHI)<br>* En entier * | 1-10 | C 07 D 257/02<br>C 08 F 12/32<br>B 01 J 45/00 |
| A | DE-A-2 803 528 (I.I. TABUSHI)<br>* En entier * | 1-10 | |
| | | | **DOMAINES TECHNIQUES RECHERCHES (Int. Cl.4)** |
| | | | C 07 D 257/00 |

Le présent rapport a été établi pour toutes les revendications

| Lieu de la recherche | Date d'achèvement de la recherche | Examinateur |
|---|---|---|
| LA HAYE | 13-07-1988 | LUYTEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)